# EUROPÄISCHE PATENTSCHRIFT

(11) **EP 3 231 464 B1**
(45) Veröffentlichungstag und Bekanntmachung des Hinweises auf die Patenterteilung: **15.07.2020**
(21) Anmeldenummer: 17165291.0
(22) Anmeldetag: 06.04.2017
(51) Int. Cl.: A61M 1/34, A61M 1/36

(54) **VORRICHTUNG ZUR EXTRAKORPORALEN BLUTBEHANDLUNG**
EXTRACORPOREAL BLOOD PROCESSING DEVICE
DISPOSITIF DESTINÉ AU TRAITEMENT DU SANG EXTRACORPOREL

(30) Priorität: 15.04.2016 DE 102016107024
(43) Veröffentlichungstag der Anmeldung: 18.10.2017
(73) Patentinhaber: B. Braun Avitum AG, 34212 Melsungen (DE)
(72) Erfinder: RIEMENSCHNEIDER, Heiko, 34327 Wagenfurth (DE)
(74) Vertreter: Winter, Brandl, Fürniss, Hübner, Röss, Kaiser, Polte - Partnerschaft mbB

(56) Entgegenhaltungen:
- EP-A1- 1 867 353
- EP-A2- 1 655 043
- WO-A1-2015/022537
- WO-A1-2016/043186
- US-A1- 2005 203 493

## Beschreibung

Die Erfindung betrifft ein Verfahren und eine Vorrichtung zur extrakorporalen Blutbehandlung, und bezieht sich insbesondere auf ein Verfahren und eine Vorrichtung zur extrakorporalen Blutbehandlung mit verzögertem Hämodiafiltrationsbehandlungsbeginn in Verbindung mit einer Regelung auf maximale Crossrate im Rahmen einer Hämodiafiltrationsbehandlung, vorzugsweise einer Online-Hämodiafiltrationsbehandlung.

Die Druckschrift WO 2015/022537 A1 beschreibt eine Blutbehandlungsvorrichtung, die Hämodialyse- und Hämodiafiltrationsverfahren durchführen kann. Während eines Hämodialyseverfahrens werden ein oder mehrere Hämodiafiltrationsverfahren in periodischen Abständen durchgeführt, um eine während der Hämodialyse gebildete Schicht aus festen Blutbestandteilen auf der Dialysemembran abzutragen und die verfügbare Membranfläche wieder zu erhöhen.

Die Druckschrift EP 3 195 890 A1 beschreibt eine Vorrichtung zur Blutreinigung, die durch entsprechende Ventilsteuerung zwischen Hämodialyse-, Hämofiltrations- und Hämodiafiltrationsverfahren umschalten kann.

Ein bekanntes Gerät für extrakorporale Blutbehandlung umfasst zumindest eine Behandlungseinheit, wie beispielsweise einen Dialysator bzw. eine Dialysatorvorrichtung, oder einen Filter, Ultrafilter oder Plasmafilter einer andersartigen Filtereinheit mit einer semipermeablen Membran, welche die Behandlungseinheit in zwei Kammern trennt. Ein extrakorporaler Blutkreislauf ermöglicht einen Fluss von aus einem Patienten entnommenem Blut durch die erste Kammer hindurch zurück zum Patienten. Gleichzeitig fließt gegenläufig dazu eine Dialysierflüssigkeit (Behandlungsflüssigkeit) durch einen geeignet ausgelegten Kreislauf über die zweite Kammer.

Das bekannte Gerät beinhaltet darüber hinaus eine Infusionsleitung für ein Substitutionsfluid, eine einlassseitig mit der zweiten Kammer verbundene Fluideinlassleitung und eine auslassseitig mit der zweiten Kammer verbundene Fluidauslassleitung. Ferner sind Sensoren vorgesehen zum Ermitteln eines ersten, sich auf das Blutvolumen eines Patienten beziehenden Parameters, eines zweiten, sich auf eine Ultrafiltrationsflussrate oder eine Gewichtsverlustrate des Patienten beziehenden Parameters, eines dritten, sich auf eine Konduktivität oder Konzentration einer die Fluidleitung und/oder die Infusionsleitung durchströmende Flüssigkeit beziehenden Parameters, und eines vierten, sich auf eine Infusionsflussrate beziehenden Parameters. Eine Steuereinheit führt eine Steuerungsprozedur zum Ausregeln einer Schwankung des Blutvolumens und eine Einstellsequenz durch zum Beaufschlagen eines Transmembrandrucks mit Werten zur Maximierung der konvektiven Austauschvorgänge.

In dem bekannten Gerät werden der Transmembrandruck und der Blutfluss ins Verhältnis gesetzt und abhängig von der Geschwindigkeit des Blutflusses und dessen Änderungen verschiedene Funktionen ausgeführt, beispielsweise ein neuer Betriebsmodus begonnen oder ein Betriebsmodus angehalten.

Bei Nierensersatzverfahren mittels Geräten der bekannten Art können verschiedenartige Komplikationen auftreten. Beispielsweise werden Systeme dazu ausgelegt, eingreifen zu können, falls es bei einer auf einen nicht optimal abgestimmten Parametereingriff durch den Anwender zurückführbaren, kalkulierbaren (zu starken) Verschlechterung des Therapieergebnisses kommen könnte.

Beispielsweise kann bei einem Anfahren der Therapie, d.h. zu einem Zeitpunkt, zu dem bereits alle Therapieparameter und die Therapieform ausgewählt sind, versucht, werden, schnellstmöglich auch einen vorbestimmten Blutfluss zu erreichen, um mit der Therapie zu beginnen. Gelingt dies nicht unmittelbar und startet die Therapie mit einem niedrigeren Blutfluss, kann es zu einer Hämokonzentration des Bluts im Dialysator kommen. Dies wird derzeit zwar systematisch überwacht, jedoch wird die Behandlung als solche unter Hinweis auf beispielsweise ein Überschreiten der Ultrafiltrationsrate/Crossrate zugelassen, wobei aufgrund bestehender Grenzwerte (30% bei einem therapeutisch angestrebten Maximalwert von 25%) nur geringer Spielraum besteht. Ein weiterer Fall entsteht in der Therapie, wenn aufgrund eines problematischen Zugangs auch nur zeitweise der Blutfluss reduziert werden muss. Der Anwender darf die verordnete Substitutionsmenge oder die Therapieart (beispielsweise von Postdilution auf Prädilution) nicht ohne Rücksprache mit dem Arzt verringern bzw. wechseln.

Ferner berücksichtigen derzeit bekannte Anordnungen die Ausbildung einer Sekundärmembrane in den ersten Minuten nicht. Komplikationen in einem Nierenersatzverfahren resultieren auch in einem Clotting (Gerinnselbildung) im extrakorporalen Kreislauf, beispielsweise aufgrund einer zu geringen Heparindosierung, eines zu niedrigen Flows und zu hoher Filtrationsraten, das sich regelmäßig in einem Anstieg des Filtereinlaufdrucks äußert. Dadurch können sich Proteine an der Innenseite des Filters ablagern und eine Abnahme der Filtrationsleistung zur Folge haben. Durch die Ablagerung kommt es zur Bildung der Sekundärmembran, der anwenderseitig durch eine ausreichende Heparinisierung, eine Prädilution oder auch die Erhöhung des Substituts zu begegnen wäre, und durch die Verringerung der Filtration (Abfall des Siebkoeffizienten) kommt es bei der Ausbildung von Sekundärmembranen zu einem Absinken des Filtrationsdruckes Pf und dadurch zu einem Anstieg des Transmembrandrucks TMP.

Da genaue Mechanismen der Entstehung der Sekundärmembranen noch nicht vollständig bekannt und darüber hinaus diese beeinflussende Maßnahmen bei der Hämofiltration wegen der Gefahr der Hämolyse nur begrenzt möglich sind, ist auch hier jeweils ebenfalls eine Rücksprache mit dem Arzt erforderlich oder wird, wie vorstehend erwähnt, bei bekannten Anordnungen in nachteiliger Weise die Sekundärmembranbildung nicht betrachtet. Es werden auch keine Prozesse automatisch durchgeführt, die die Querflussrate bzw. Querrate oder "Crossrate" bewerten und einen Anwendungsfehler verhindern. Der Anwender muss daher sämtliche Folgen aus einer Warnmeldung des Geräts oder Systems sofort richtig interpretieren, kann sich aber dennoch für nur zwei nicht optimale Vorgehensweisen entscheiden. Einerseits kann er die Therapie unterbrechen, bis der Blutfluss wieder adäquat ist. Dadurch verzögert sich jedoch automatisch auch das Behandlungsende. Andererseits kann er die Membranbelastung und die damit einhergehende Effektivitätsminderung und in der Folge ein vermindertes Therapieergebnis in Kauf nehmen.

Der Erfindung liegt daher als eine Aufgabe zugrunde, ein Verfahren und eine Vorrichtung zur extrakorporalen Blutbehandlung bereitzustellen, die die vorstehenden Nachteile und Probleme überwinden und einen Anwender automatisch unterstützen und in kritischen Situationen korrekt reagieren.

Darüber hinaus sollen aus beispielsweise einer temporären
Zugangsproblematik resultierende Anwendungsfehler erkennbar und bis zum Erreichen eines adäquaten und ausreichenden Blutflusses kompensierbar sein.

Diese Aufgabe wird erfindungsgemäß gelöst durch eine Vorrichtung zur extrakorporalen Blutbehandlung mit den Merkmalen des Anspruchs 1. Vorteilhafte Weiterbildungen der Erfindung sind Gegenstand der beigefügten Unteransprüche.

Der Erfindung liegt als eine allgemeine Idee zugrunde, im vorgenannten Rahmen eine reine Konstantgrößenregelung des Substitutionsflusses im Hinblick auf eine maximale Leistung eines Dialysators einhergehend mit einem automatisch verzögerten Beginn einer Hämodiafiltration in vorzugsweise der Behandlungsart Hämodiafiltration bzw. HDF (Postdilution) bzw. "post HDF" oder HDF (post) bereitzustellen, wodurch Stressbelastungen reduziert und ein Therapieziel besser erreichbar werden. In anderen Worten soll durch eine spezielle Regelung ein verzögerter Online-HDF-Therapiestart nach ausreichender Ausbildung der Sekundärmembran und eine automatisierte Gewährleistung einer maximal möglichen Dialysatorleistung über den Therapieverlauf hinweg ein maximal mögliches Therapieergebnis sichergestellt werden. Hierzu wird die Konstantgrößenregelung des Substitutionsflusses durchgeführt, die einem Arzt Vorberechnungen erspart und einem Anwender, beispielsweise einer Schwester, Sicherheit gibt, dass die Behandlung fortwährend in einem maximal möglichen physiologischen Bereich erfolgt. Die Ausbildung der Sekundärmembrane und das Anfahren der HDF (mit Postdilution) mit der richtigen (Substitutions-)Menge erfolgt ebenso automatisch und sicher zur jeweiligen Anwendung passend.

Die Regelung bzw. Steuerung implementiert ein einfaches und sicheres Verfahren, um dem Anwender in kritischen Situationen die jeweils benötigte Zeit zu verschaffen, bis beispielsweise ein behandelnder Arzt oder leitendes Personal notwendige Therapieänderungen durchführen kann. Das Verfahren und die Vorrichtung verhindern Anwendungsfehler und minimieren das Risiko eines durch zu hohe Crossraten verblockenden Dialysators in der post HDF-Behandlung. Es ist nicht weiter erforderlich, auf eine ausreichende Ausbildung der Sekundärmembran zu achten, sondern der Anwender kann bereits im Vorfeld bzw. während der Vorbereitung sämtliche entsprechenden Parameter der Therapie anwählen, da ein Zeitpunkt der Umschaltung von HD auf Online-HDF und eine Substitutionsmenge automatisch berechnet und zugeschaltet werden.

Ein das Verfahren durchführendes und die Vorrichtung beinhaltendes System unterstützt somit den Anwender und überwacht absichernd den Prozessablauf. Infundierte Mengen werden ebenso kontinuierlich angezeigt wie zu erwartende, mit einer jeweils momentanen Einstellung mögliche bzw. erreichbare Größen. Ein patientenbezogener Wert einer Proportionalmenge kann jederzeit individuell eingestellt werden, so dass sich der Anwender vorteilhaft wieder anderweitig wichtigen Behandlungsparametern zuwenden kann. Der Arzt legt eine maximale Crossrate patientenbezogen fest. Das System steuert sodann selbstständig sämtliche erforderlichen Parameter. In anderen Worten wird anstelle einer Zielmenge (Substitution) nur noch eine patientenbezogene maximale Proportion der Substitution in einem festen Verhältnis zum Blutfluss verwendet. Damit wird risikolos das mögliche Maximum an Therapieeffektivität erreicht, und entfallen bislang technisch bedingte Vorberechnungen durch den Arzt.

Im Einzelnen werden die vorstehenden Vorteile realisiert und die Aufgabe gelöst durch eine Vorrichtung zur extrakorporalen Blutbehandlung, die eine Dialysatorvorrichtung aufweist und zur Durchführung einer Hämodialyse und einer Hämodiafiltration mit Postdilution ausgelegt ist, wobei die Vorrichtung zur extrakorporalen Blutbehandlung die folgenden Merkmalen aufweist: eine anwenderseitig bedienbare und/oder ablesbare Benutzerschnittstelle zum Beginnen der Blutbehandlung mittels Hämodialyse auf der Grundlage von Vorgabewerten für die Hämodialyse; zumindest eine Erfassungs- und/oder Recheneinheit zum Ermitteln aktueller Werte und/oder Verhältnisse zumindest eines Blutflusses, einer Ultrafiltrationsmenge, einer Substitutionsmenge und/oder einer Substitutionsart; eine Sensoreinrichtung zum Erfassen eines Therapiefortschritts auf der Grundlage eines Ausgangssignals einer Sensoreinrichtung; eine Ermittlungseinrichtung zum Ermitteln einer Ultrafiltrationsrate in der Dialysatorvorrichtung; eine Ermittlungseinrichtung zum Ermitteln eines Ausbildungszeitpunkts einer Sekundärmembran in der Dialysatorvorrichtung auf der Grundlage der ermittelten Ultrafiltrationsrate; eine Umschalteinrichtung zum Wechseln von der Hämodialyse auf eine Hämodiafiltration mit Postdilution nach Verstreichen einer vorbestimmten Zeitspanne; und eine Regeleinrichtung zum Regeln der Substitutionsmenge während der Hämodiafiltration mit Postdilution.

Die vorbestimmte Zeitspanne ist eine Zeitspanne nach einer ersten Erfassung eines Blutflusses an der Sensoreinrichtung. Der Wechsel von der Hämodialyse auf die Hämodiafiltration mit Postdilution erfolgt automatisch, falls zu der vorbestimmten Zeit eine vorbestimmte Crossrate ermittelt wird, wobei bei der vorbestimmten Crossrate ein vorbestimmter Behandlungsblutfluss als erreicht und die Sekundärmembran als einen vorbestimmten Ausbildungszustand aufweisend angenommen wird.

Bevorzugt beträgt die vorbestimmte Zeitspanne initial etwa 3 bis 5 Minuten und ist die Vorrichtung und/oder das Verfahren dazu ausgelegt, nach Beginn der Behandlung eine tatsächliche Zeitspanne auf der Grundlage von Vorgabewerten und aktuell berechneten Werten anzupassen und die Hämodiafiltration mit Postdilution automatisch verzögert anzufahren.

Bevorzugt erfolgt die erste Erfassung des Blutflusses mittels eines an der Luftdetektoreinrichtung angeordneten Blutsensors als der Sensoreinrichtung und wird der angenommene Ausbildungszustand der Sekundärmembran aus der ermittelten Crossrate abgeleitet.

Bevorzugt wird die Substitutionsmenge durch die Regeleinrichtung in Übereinstimmung mit einer Vorgabe einer Anwendung geregelt, wobei die Substitutionsmenge einen vorbestimmten maximalen prozentualen Anteil des Blutflusses nicht überschreitet.

Bevorzugt wird die Substitutionsmenge durch die Regeleinrichtung auf eine konstante Größe oder Menge geregelt.

Bevorzugt beträgt der vorbestimmte maximale prozentuale Anteil 25 bis 30% des Blutflusses.

Bevorzugt wird die Substitutionsmenge durch die Regeleinrichtung automatisch reduziert, wenn der Blutfluss eine vorbestimmte Mindestmenge unterschreitet, und wird eine über die reduzierte Substitutionsmenge benachrichtigende Meldung für einen Anwender ausgegeben.

Bevorzugt wird bei Erreichen einer vorbestimmten Crossrate die Substitutionsmenge durch die Regeleinrichtung automatisch begrenzt und eine über die Begrenzung der Substitutionsmenge benachrichtigende Meldung für einen Anwender ausgegeben.

Bevorzugt sind sämtliche erforderliche Parameter anwenderseitig vorgebbar und ist die Vorrichtung und/oder das Verfahren dazu ausgelegt, Maximalmengen der anwenderseitig vorgegebenen Parameter erforderlichenfalls selbsttätig zu verringern.

Die Erfindung wird nachstehend anhand eines bevorzugten Ausführungsbeispiels unter Bezugnahme auf die beigefügte Zeichnung näher beschrieben. Es zeigen:
Fig. 1 eine schematische Darstellung eines in einem Ausführungsbeispiel des Verfahrens und der Vorrichtung zur extrakorporalen Blutbehandlung angeordneten Blutkreislauf mit Postdilution und/oder Prädilution bei Online-Hämodiafiltration (Online-HDF);
Fig. 2 eine schematische Darstellung zur Erläuterung der Ausbildung einer Sekundärmembran in einer Dialysatorvorrichtung;
Fig. 3 eine schematische Darstellung zur Veranschaulichung von Flussverhältnissen bei Online-HDF im Inneren eines Dialysators; und
Fig. 4 schematisch und stark vereinfacht ein Ablaufdiagramm des Verfahrens zur extrakorporalen Blutbehandlung gemäß einem Ausführungsbeispiel

In der nachfolgenden Figurenbeschreibung können in einzelnen Figuren gleiche oder gleichwirkende Elemente und/oder Komponenten gleich und/oder mit denselben Bezugszeichen bezeichnet und zweckmäßig nicht redundant beschrieben sein. In Fällen, in welchen ein nachfolgendes Ausführungsbeispiel funktionell zumindest einem vorangehenden entspricht, d.h. entsprechende Funktionen, Anordnungen und/oder Verfahrens- oder Betriebsabläufe gleichermaßen umfasst sind, wird zweckmäßig nur auf Unterschiede eingegangen.

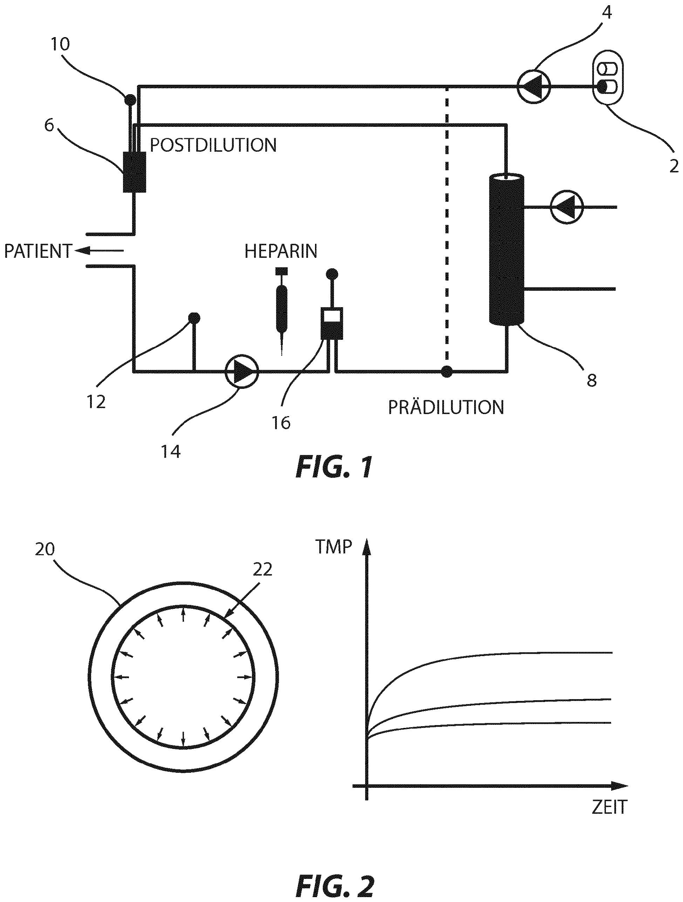

Fig. 1 zeigt eine schematische Darstellung eines in einem Ausführungsbeispiel des Verfahrens und der Vorrichtung zur extrakorporalen Blutbehandlung verwendeten bzw. angeordneten Blutkreislaufs mit Postdilution und/oder Prädilution bei Online-Hämodiafiltration (Online HDF).

Ein Gerät zur extrakorporalen Blutbehandlung, wie beispielsweise eine Dialysemaschine zur Reinigung von Blut eines Patienten, wenn z.B. dessen Nierenfunktion eingeschränkt oder eingestellt ist, weist einen Dialysator auf, der einerseits von dem zu reinigenden Blut des Patienten und andererseits von einer Dialysierflüssigkeit oder Dialyselösung, vorzugsweise gemäß dem Gegenstromprinzip, durchströmt wird, wobei gewisse gelöste Substanzen (z.B. Harnstoff) aus dem Blut an die Dialysierflüssigkeit übergehen.

Das Gerät zur extrakorporalen Blutbehandlung weist ferner einen arteriellen Blutkreislauf, in welchem eine Blutpumpe angeordnet ist, einen venösen Blutkreislauf, eine Dialysierflüssigkeitszufuhrleitung, die dem Dialysator frische Dialysierflüssigkeit zuführt, eine Dialysierflüssigkeitsabführleitung, die verbrauchte Dialysierflüssigkeit aus dem Dialysator abführt bzw. ausleitet, eine Substitutionsleitung und eine Substitutionspumpe, die in der Substitutionsleitung fließendes Substituat (Elektrolytlösung) einem vor (Prädilution) oder nach (Postdilution) dem Dialysator liegenden Dilutionspunkt in dem arteriellen und/oder dem venösen Blutkreislauf zuführt. Eine weiter bereitgestellte Steuereinrichtung misst die Konzentration von Blut in beispielsweise einem vorbestimmten Dilutionskanalabschnitt und steuert die von der Substitutionspumpe zur Verhinderung zu starker Bluteindickung und zum kontinuierlichen Ausgleich von über den notwendigen Flüssigkeitsentzug hinausgehender Ultrafiltration und damit Vermeidung von Volumenverlusten zugeführte Substituatmenge auf der Grundlage der gemessenen oder einer abgeschätzten Blutkonzentration. Ein Gerät zur extrakorporalen Blutbehandlung der vorgenannten Art kann dazu ausgelegt sein, Substituat nach dem Prinzip der Online-Hämodiafiltration (Online-HDF) oder der Online-Hämofiltration vorzubereiten, zuzuführen und zu substituieren. Bei einem Online-HDF-Verfahren wird die Substitutionsflüssigkeit bzw. das Substituat aus der Dialysierflüssigkeit gewonnen und entweder vor oder nach dem Filter infundiert.

Systematik, Aufbau, Komponenten und Funktionsweise des vorgenannten Geräts zur extrakorporalen Blutbehandlung sind an sich grundlegend bekannt und werden daher diesbezüglich hierin einbezogen und nicht weiter beschrieben.

Der in Fig. 1 gezeigte Blutkreislauf mit Postdilution und/oder Prädilution bildet Teil eines Geräts zur extrakorporalen Blutbehandlung wie vorstehend beschrieben. Aus einer online, d.h. Substituat während einer laufenden Therapie im oder am Gerät zur extrakorporalen Blutbehandlung erzeugenden Substituatquelle 2 stammendes Substituat wird von einer Online-Substituatpumpe 4 durch eine Dilutionsleitung zu einer Luftdetektoreinrichtung transportiert. In die Luftdetektoreinrichtung mündet eine von einem Dialysator 8, in diesem Ausführungsbeispiel ein High-flux-Dialysator, kommende venöse Blutleitung. Ferner ist ein venöser Druckaufnehmer 10 mit der Luftdetektoreinrichtung verbunden. In den Dialysator 8 mündet eine von einem Patienten kommende arterielle Blutleitung, in deren Verlauf ein arterieller Druckaufnehmer 12, eine arterielle Blutpumpe 14, ein weiterer Druckaufnehmer 16 an einer arteriellen Expansionskammer vor dem Dialysator 8 und ein Einleitungspunkt für eine Prädilution angeordnet sind. Des Weiteren kann arteriell auch eine von dem Gerät gesteuerte Heparinzugabe (Gerinnungshemmer) zur Verhinderung der Blutgerinnung vorgesehen sein.

Fig. 2 zeigt eine schematische Darstellung zur Erläuterung der Ausbildung einer Sekundärmembran in einer Dialysatorvorrichtung. Eine Sekundärmembran entsteht als Diffusionsschicht 22 an den Hohlmembranen 20 in dem Dialysator 8 während einer laufenden Blutbehandlung durch Anlagern von beispielsweise zellulären Blutbestandteilen und Proteinen bzw. Plasmaproteinen an die Dialysatormembran. Die Bildung einer Sekundärmembran ist mit dem Transmembrandruck TMP gekoppelt. Der Transmembrandruck TMP im Dialysator 8 ist ein hydrostatisches Druckgefälle zwischen Blutseite und Dialysierflüssigkeitsseite, der von der Ultrafiltration genutzt wird.

Die Funktion und die Integrität des Dialysators 8 können maschinenseitig durch Messung der dynamischen Eingangs- und Ausgangsdrücke überwacht werden. Beispielsweise kann unter der Annahme eines linearen Druckabfalls aus den Eingangsdrücken und Ausgangsrücken ein mittlerer Druck für die Blutseite und ein mittlerer Druck für die Dialysierflüssigkeitsseite bestimmt werden, deren Differenz in einem mittleren Transmembrandruck TMP resultiert. Dieser mittlere Transmembrandruck TMP kann sodann für eine Überwachung, ob der Filter durch Ablagerungen und Bildung einer Sekundärmembran verstopft ist, herangezogen werden.

Wie rechtsseitig in Fig. 2 dargestellt, steigt der Transmembrandruck TMP zu Beginn einer HDF-Dialyse und während der Ausbildung einer Sekundärmembran zunächst an und erreicht dann zumindest näherungsweise ein Plateau.

Obwohl mit dem zu Beginn ansteigenden Transmembrandruck TMP ein Verblocken oder Verstopfen der Poren des Dialysators durch beispielsweise sich anlagernde Proteine einhergeht und dies praktisch einer Verringerung der Filteroberfläche entspricht, wodurch sich die Effizienz der gesamten Therapie verringern kann, ergeben sich aufgrund der Ausbildung der Sekundärmembran bzw. Diffusionsschicht auch Vorteile, wie beispielsweise eine Unterdrückung der Komplementaktivierung, eine Verringerung der Thrombogenizität, eine Unterdrückung der Thrombozytenaktivierung und einer Verringerung der Proteinabsorption.

In dem vorliegenden Ausführungsbeispiel wird davon ausgegangen, dass sich eine ausreichende Sekundärmembran nach etwa 3 bis 5 Minuten nach Therapiebeginn bei geringem Blutfluss und minimaler Ultrafiltration ausgebildet hat.

In einem beispielsweisen Fall kann sich zu Beginn der HDF-Dialyse bei einem Dialysierflüssigkeitsfluss von etwa 30% des Blutflusses und Postdilution dialysatoreingangsseitig ein Hämatokritgehalt von etwa 35% und dialysatorausgangsseitig ein Hämatokritgehalt von etwa 50% einstellen. Aufgrund der Ausbildung der Sekundärmembran bzw. mit dem Anstieg des Transmembrandrucks TMP einhergehend können sich diese Anteile bei einer beispielsweise angenommenen Verringerung des Blutvolumens um 15% auf dialysatoreingangsseitig etwa 41% und dialysatorausgangsseitig etwa 59% verändern. Der Anstieg des Hämatokrits ist umgekehrt proportional zur Veränderung des Blutvolumens, d.h. die Abnahme des relativen Blutvolumens ist gekennzeichnet durch den Anstieg des Hämatokritgehalts. Durch die Messung des Hämatokritgehalts kann eine relative Veränderung des (relativen) Blutvolumens erfasst werden.

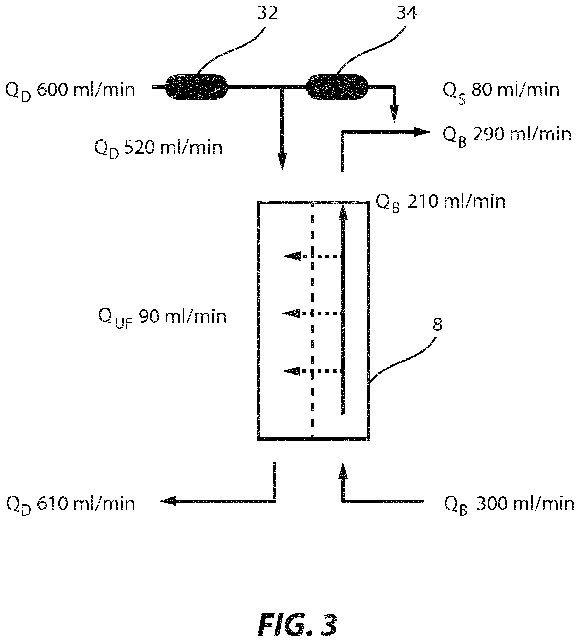

Fig. 3 zeigt eine schematische Darstellung zur Veranschaulichung von Flussverhältnissen bei Online-HDF im Inneren eines Dialysators, beispielsweise des Dialysators 8 gemäß Fig. 1. Ferner sind in Fig. 3 mit 32 ein DF-Filter bzw. Dialysierflüssigkeitsfilter als eine erste Filterstufe und mit 34 ein HDF-Filter als eine zweite Filterstufe im Dialysierflüssigkeitsfluss bezeichnet, die ebenfalls in dem Gerät zur extrakorporalen Blutbehandlung angeordnet sind. Darüber hinaus bezeichnet Q_{D} einen Dialysierflüssigkeitsfluss, bezeichnet Q_{B} einen Blutfluss, bezeichnet Q_{UF} einen Ultrafiltrationsfluss und bezeichnet Qs einen Substituatfluss für die Prä- oder Postdilution.

Wie in Fig. 3 dargestellt, wird der gesamte ursprüngliche Dialysierflüssigkeitsfluss, in dem vorliegenden Ausführungsbeispiel beispielsweise Q_{D} = 600 ml/min, durch den DF-Filter 32 geleitet. Nach dem DF-Filter 32 wird ein Teil des Dialysatflusses Q_{D}, beispielsweise ein Anteil von 520 ml/min, durch den Dialysator 8 geleitet. Der verbleibende Teil des Dialysierflüssigkeitsflusses Q_{D} wird zusätzlich durch den HDF-Filter 34 geführt und nach diesem als Substituatfluss Qs = 80 ml/min zur Prä- oder Postdilution bereitgestellt.

Gegenstromig zu dem Dialysierflüssigkeitsfluss durch den Dialysator 8 durchströmt diesen der Blutfluss Q_{B}, der in dem vorliegenden Ausführungsbeispiel beispielsweise dialysatoreingangsseitig Q_{B} = 300 ml/min und dialysatorausgangsseitig noch Q_{B} = 210 ml/min beträgt. Hieraus errechnet sich in diesem Ausführungsbeispiel eine Crossrate oder Crossrate der Ultrafiltration von Q_{UF} = 90 ml/min oder etwa 27%. In anderen Worten treten Q_{UF} = 90 ml/min Flüssigkeit durch die Membran des Dialysators 8 hindurch (konvektiver Transport). Wie rechtsseitig des HDF-Filters angedeutet, wird der Substituatfluss Q_{S} = 80 ml/min dem dialysatorausgangsseitigen Blutfluss Q_{B} = 210 ml/min postdilutiv hinzugefügt, so dass ein venös abströmender Blutfluss Q_{B} = 290 ml/min resultiert.

Da bei Postdilution das Verhältnis von Blut zu gesamter Ultrafiltration maximal 25 bis 30% betragen sollte, und gleichzeitig das gesamte Substitutionsvolumen etwa ein Drittel des Körpergewichts des Patienten betragen sollte, ergeben sich für die Postdilution in einem Beispielfall für einen 81 kg schweren Patienten die folgenden rechnerischen Werte:

| | |
|---|---|
| Körpergewicht: | 81 kg |
| Gewichtsverlust: | 3 kg |
| Behandlungszeit: | 5 Stunden |

30% von 81 kg = 27 l Substitutionsvolumen
27 l Substitutionsvolumen + 3 l Gewichtsverlust = 30 l Nettoultrafiltrationsmenge 30 l in 5 Stunden Behandlungszeit ergibt eine Ultrafiltrationsrate von 6 l/h oder 100 ml/min. Die gesamte Ultrafiltrationsmenge beträgt mithin 3 l, und der Blutfluss Q_{B} sollte wenigstens etwa 350 ml/min betragen.

In Übereinstimmung mit der vorstehenden Beschreibung wird bei dem Verfahren und der Vorrichtung zur extrakorporalen Blutbehandlung des vorliegenden Ausführungsbeispiels ein verzögerter Hämodiafiltrationsbehandlungsbeginn in Verbindung mit einer Regelung auf maximale Crossrate der Ultrafiltration im Rahmen einer Hämodiafiltrationsbehandlung, vorzugsweise einer Online-Hämodiafiltrationsbehandlung, durchgeführt.

Dazu werden vor Beginn der Behandlung und wie vorstehend beispielhaft anhand praxisnaher Größenordnungen angedeutet zunächst anwendbare Verhältnisse aus Blutfluss, Ultrafiltrationsmenge, Substitutionsmenge und Substitutionsart ermittelt oder berechnet.

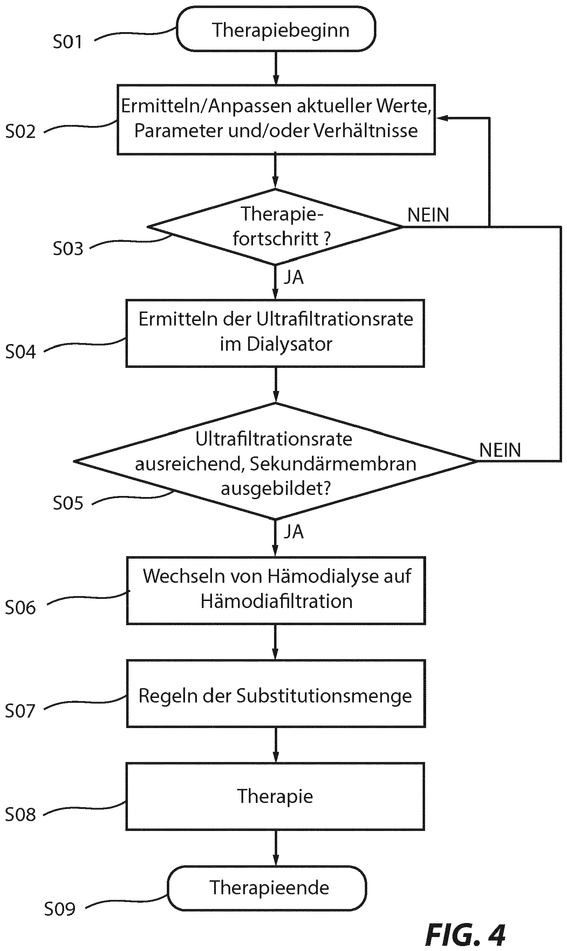

Fig. 4 zeigt schematisch ein stark vereinfachtes Ablaufdiagramm des Verfahrens. Nach dem Ermitteln der erforderlichen Behandlungs- oder Therapieparameter und der Auswahl oder Festlegung der Therapieform wird in einem Schritt S01 die Therapie von einem Anwender, beispielsweise einer Schwester in einer Dialysestation, mittels Hämodialyse auf der Grundlage der Vorgabewerte für die Hämodialyse angefahren. Daraufhin schreitet das Verfahren zu einem Schritt S02 fort.

In Schritt S02 werden aktuelle Werte bzw. Parameter und/oder Verhältnisse zumindest des Blutflusses, der Ultrafiltrationsmenge, der Substitutionsmenge und/oder der Substitutionsart ermittelt oder angepasst. Die ermittelten Werte sind über die Benutzeroberfläche des Geräts zur extrakorporalen Blutbehandlung änderbar. Der Anwender versucht in dieser Phase, möglichst schnell den vorbestimmten Blutfluss zu erreichen, um mit der eigentlichen Therapie beginnen zu können. Dabei kann der Anwender zwar alle Werte und Parameter eingeben, jedoch werden systemseitig Maximalmengen erforderlichenfalls geeignet reduziert. Das Verfahren schreitet zu einem nächsten Schritt S03 fort.

In Schritt S03 wird der Therapiefortschritt auf der Grundlage eines Ausgangssignals einer Sensoreinrichtung 6 erfasst. Beispielsweise kann der Therapiefortschritt durch Erfassen des Eintreffens einer ersten Blutmenge mittels eines Blutsensors in oder an der Luftdetektoreinrichtung erfasst werden. Falls ein ausreichender Therapiefortschritt erfasst wird (JA in Schritt S03), schreitet das Verfahren zu einem nächsten Schritt S04 fort. Falls die Therapie noch nicht weit genug fortgeschritten ist (NEIN in Schritt S03), kehrt das Verfahren zu Schritt S02 zurück.

In Schritt S04 wird die Ultrafiltrationsrate bzw. der Ultrafiltrations-Querfluss durch die Membran in der Dialysatoreinrichtung 8 ermittelt. In anderen Worten wird eine Crossratenberechnung durchgeführt. Sodann schreitet das Verfahren zu einem Schritt S05 fort.

In Schritt S05 wird geprüft, ob eine mit den übrigen Parametern stimmige Crossrate, d.h. der gewünschte Behandlungsblutfluss erreicht ist, und ob die Sekundärmembran weit genug ausgebildet ist. Ist der Behandlungsblutfluss erreicht und die Sekundärmembran weit genug ausgebildet (JA in Schritt S05), schreitet das Verfahren zu einem Schritt S06 fort. Falls der Behandlungsblutfluss noch nicht erreicht und/oder die Sekundärmembran noch nicht weit genug ausgebildet ist (NEIN in Schritt S05), kehrt das Verfahren zu Schritt S02 zurück. Alternativ kann vorgesehen sein, dass das Verfahren zu Schritt S04 zurückkehrt.

In Schritt S06 erfolgt ein verzögertes Anfahren der Hämodiafiltration mit Postdilution bzw. HDF(post), welches automatisch gemäß Vorgaben und Berechnung durchgeführt wird. Das automatische Anfahren der HDF (post), d.h. der Wechsel von der anfänglichen Hämodialyse auf die Hämodiafiltration, ist in der Praxis etwa 5 Minuten nach dem ersten Kontakt mit Blut an der Luftdetektoreinrichtung zu erwarten, sofern die eine geeignete Crossrate erreicht worden ist, d.h. der Behandlungsblutfluss erreicht ist und die Sekundärmembrane weit genug ausgebildet ist. Sodann schreitet das Verfahren zu einem Schritt S07 fort.

In Schritt S07 wird die Substitutionsmenge, d.h. die Menge des mittels Postdilution zugeführten Substituats, nach Vorgabe der Anwendung und auf maximal 25% bis 30% des Blutflusse geregelt.

Beispielsweise kann die Substitutionsmenge auf eine konstante Größe geregelt werden, die maximal 25% bis 30% des Blutflusses beträgt, sofern der Blutfluss konstant bleibt, oder auf eine Größe geregelt werden, die konstant maximal 25 bis 30% des Blutflusses beträgt, wenn sich der Blutfluss verändert. Bei einem sich verändernden und dann gegebenenfalls nicht ausreichenden Blutfluss ist vorgesehen, dass die Substitutionsmenge ebenfalls entsprechend reduziert wird, um den vorgenannten prozentualen Anteil beizubehalten. In diesem Fall sind das Verfahren und die Vorrichtung dazu konfiguriert, beispielsweise über die Benutzeroberfläche des Geräts oder dergleichen einen Hinweise für den Anwender darauf hin auszugeben, dass die Substitutionsmenge automatisch reduziert wurde.

Nach Schritt S07 wird in einem stark vereinfacht dargestellten Schritt S08 die weitere Therapie oder Blutbehandlung durchgeführt, und endet danach das Verfahren bzw. dessen Prozess(e) bei Therapieende in einem Schritt S09.

Wie vorstehend beschrieben wurde, wird erfindungsgemäß die maximale Leistung des Dialysators genutzt und gehalten. Wenn es durch beispielsweise einen möglicherweise nicht optimal abgestimmten Parametereingriff eines Anwenders zu einer kalkulierbaren (massiven) Verschlechterung des Therapieergebnisses kommen könnte, kann systemseitig eingegriffen werden. Ursachen für systemseitige Eingriffe können vielfältig sein und beispielsweise auf die Komplexität und Vielfalt von zunehmend Expertenwissen bedingenden Anwendungsarten, einen jeweiligen Personalschlüssel und die Qualifikation von Bedienpersonal, eine nicht unmittelbare Erreichbarkeit des zuständigen Arztes zurückzuführen und Gründe für ein vermindertes Therapieergebnis sein.

Das erfindungsgemäße Verfahren und die erfindungsgemäße Vorrichtung unterstützen den Anwender bei Anwendungen, die die Leistung des Dialysators unmittelbar beeinträchtigen. Notwendige Parameter werden dabei automatisch korrigiert. Zusätzlich wird auch ein Hinweis ausgegeben, jedenfalls aber wird in einem derartigen Fall aufgrund der erfindungsgemäßen Beschränkung der Substitution auf einen maximalen Faktor das Leistungsvermögen des Dialysators nicht beeinträchtigt. Aufgrund der Beschränkung auf einen maximalen Faktor, d.h. der Konstantgrößenregelung des Substitutionsflusses, wird der Dialysator nicht mehr grenzwertüberschreitend belastet.

Bei Erreichen der Crossrate wird die Substitutionsmenge automatisch begrenzt und der Anwender wird hierzu informiert. Beispielsweise kann eine Anzeige oder Meldung wie folgt ausgegeben werden: "Achtung: Die infundierte Menge wird gemindert, das therapeutische Ziel kann unter Umständen nicht mehr erreicht werden." Die Substitutionszielmenge kann der Anwender jederzeit verändern. Ebenso kann er Zielwarnungen jederzeit einschalten und ausschalten.

Es versteht sich, dass die Erfindung nicht auf das beschriebene Ausführungsbeispiele und dessen Modifikationen beschränkt ist, sondern dass sich innerhalb des durch die nachstehenden Ansprüche definierten Schutzumfangs für den Fachmann gleichwohl naheliegend Kombinationen von zumindest Teilen dieser Ausführungsbeispiele, Modifikationen und Äquivalente ergeben können.

## Patentansprüche

1. Vorrichtung zur extrakorporalen Blutbehandlung, die eine
Dialysatorvorrichtung (8) aufweist und zur Durchführung einer Hämodialyse und einer Hämodiafiltration mit Postdilution ausgelegt ist, mit:
- einer anwenderseitig bedienbaren und/oder ablesbaren Benutzerschnittstelle, die eingerichtet ist, die Blutbehandlung mittels Hämodialyse auf der Grundlage von Vorgabewerten für die Hämodialyse zu beginnen;
- zumindest einer Erfassungs- und/oder Recheneinheit, die eingerichtet ist, aktuelle Werte und/oder Verhältnisse zumindest eines Blutflusses, einer Ultrafiltrationsmenge, einer Substitutionsmenge und/oder einer Substitutionsart zu ermitteln;
- einer Sensoreinrichtung (6), die eingerichtet ist, einen Therapiefortschritt auf der Grundlage eines Ausgangssignals einer Sensoreinrichtung (6) zu erfassen;
- einer Ultrafiltrationsraten-Ermittlungseinrichtung, die eingerichtet ist, eine Ultrafiltrationsrate in der Dialysatorvorrichtung (8) zu ermitteln; und
- eine Regeleinrichtung, die eingerichtet ist, die Substitutionsmenge während der Hämodiafiltration mit Postdilution zu regeln,
**dadurch gekennzeichnet, dass**
die Vorrichtung zur extrakorporalen Blutbehandlung weiter aufweist:
- eine Sekundärmembran-Ermittlungseinrichtung, die eingerichtet ist, einen Ausbildungszeitpunkt einer Sekundärmembran in der Dialysatorvorrichtung (8) auf der Grundlage der ermittelten Ultrafiltrationsrate zu ermitteln; und
- eine Umschalteinrichtung, die eingerichtet ist, von der Hämodialyse auf eine Hämodiafiltration mit Postdilution nach Verstreichen einer vorbestimmten Zeitspanne zu wechseln, wobei die vorbestimmte Zeitspanne eine Zeitspanne nach einer ersten Erfassung eines Blutflusses an der Sensoreinrichtung (6) ist, und den Wechsel von der Hämodialyse auf die Hämodiafiltration mit Postdilution automatisch vorzunehmen, falls die Ultrafiltrationsraten-Ermittlungseinrichtung nach Verstreichen der vorbestimmten Zeitspanne eine vorbestimmte Ultrafiltrationsrate ermittelt, bei der ein vorbestimmter Behandlungsblutfluss als erreicht gilt und die Sekundärmembran als einen vorbestimmten Ausbildungszustand aufweisend gilt.

2. Vorrichtung nach Anspruch 1, wobei die vorbestimmte Zeitspanne initial etwa 3 bis 5 Minuten beträgt und die Vorrichtung dazu ausgelegt ist, nach Beginn der Behandlung die vorbestimmte Zeitspanne auf eine tatsächliche Zeitspanne auf der Grundlage von Vorgabewerten und aktuell berechneten Werten anzupassen und die Hämodiafiltration mit Postdilution automatisch verzögert anzufahren.

3. Vorrichtung nach Anspruch 1, wobei die Sensoreinrichtung (6) eingerichtet ist, die erste Erfassung des Blutflusses mittels eines an einer Luftdetektoreinrichtung angeordneten Blutsensors vorzunehmen.

4. Vorrichtung nach einem der vorangehenden Ansprüche, wobei die Regeleinrichtung eingerichtet ist, die Substitutionsmenge in Übereinstimmung mit einer patientenbezogenen maximalen Proportion der Substitution in einem festen Verhältnis zum Blutfluss zu regeln.

5. Vorrichtung nach einem der vorangehenden Ansprüche, wobei die Regeleinrichtung eingerichtet ist, die Substitutionsmenge auf eine konstante Größe oder Menge zu regeln.

6. Vorrichtung nach Anspruch 4, wobei das Verhältnis zum Blutfluss maximal 25 bis 30% des Blutflusses beträgt.

7. Vorrichtung nach einem der Ansprüche 4 bis 6, wobei die Regeleinrichtung eingerichtet ist, die Substitutionsmenge automatisch zu reduzieren, wenn der Blutfluss eine vorbestimmte Mindestmenge unterschreitet, und eine über die reduzierte Substitutionsmenge benachrichtigende Meldung für einen Anwender auszugeben.

8. Vorrichtung nach einem der vorangehenden Ansprüche, wobei die Regeleinrichtung eingerichtet ist, bei Erreichen einer vorbestimmten Ultrafiltrationsrate die Substitutionsmenge automatisch zu begrenzen und eine über die Begrenzung der Substitutionsmenge benachrichtigende Meldung für einen Anwender auszugeben.

9. Vorrichtung nach einem der vorangehenden Ansprüche, wobei sämtliche erforderliche Parameter anwenderseitig vorgebbar sind und die Vorrichtung dazu ausgelegt ist, Maximalmengen der anwenderseitig vorgegebenen Parameter erforderlichenfalls selbsttätig zu verringern.

## Claims

1. A device for extracorporeal blood treatment comprising a dialyzer device (8) and designed for the performance of a hemodialysis and a hemodiafiltration with post-dilution, comprising:
- a user interface to be operated and/or read by a user configured for starting the blood treatment by means of hemodialysis on the basis of default values for the hemodialysis;
- at least one recording and/or computing unit configured for determining current values and/or ratios of at least one blood flow, an ultrafiltration quantity, a substitution quantity and/or a type of substitution;
- a sensor means (6) configured for recording a therapy progress on the basis of an output signal of a sensor means (6);
- an ultrafiltration-rate determination means configured for determining an ultrafiltration rate in the dialyzer device (8); and
- a regulating means configured for regulating the substitution quantity during hemodiafiltration with post-dilution,
**characterized in that**
the device for extracorporeal blood treatment furthermore comprises:
- a secondary-membrane determination means configured for determining a time of formation of a secondary membrane in the dialyzer device (8) on the basis of the determined ultrafiltration rate; and
- a switching means configured for changing from hemodialysis to a hemodiafiltration with post-dilution after a predetermined period of time has elapsed, wherein the predetermined period of time is a period of time after a first recording of a blood flow at the sensor means (6), and
the change from hemodialysis to hemodiafiltration with post-dilution is performed automatically, if the ultrafiltration-rate determination means determines a predetermined ultrafiltration rate after the predetermined period of time has elapsed, at which a predetermined treatment blood flow is assumed as having been achieved and the secondary membrane is assumed as comprising a predetermined state of formation.

2. The device according to claim 1, wherein the predetermined period of time is initially approximately 3 to 5 minutes, and the device is designed to adjust, after the start of treatment, the predetermined period of time to an actual period of time on the basis of default values and currently calculated values, and to start hemodiafiltration with post-dilution in an automatically delayed manner.

3. The device according to claim 1, wherein the sensor means (6) is configured to perform the first recording of the blood flow by means of a blood sensor arranged on an air detector means (6).

4. The device according to one of the preceding claims, wherein the regulating means is configured to regulate the substitution quantity in accordance with a patient-related, maximum proportion of substitution in a fixed ratio to the blood flow.

5. The device according one of the preceding claims, wherein the regulating means is configured to regulate the substitution quantity to a constant value or quantity.

6. The device according to claim 4, wherein the ratio to the blood flow maximally is 25 to 30 % of the blood flow.

7. The device according to one of claims 4 to 6, wherein the regulating means is configured to automatically reduce the substitution quantity, when the blood flow falls below a predetermined minimum quantity, and issues a message for a user informing about the reduced substitution quantity.

8. The device according one of the preceding claims, wherein the regulating means is configured to automatically limit the substitution quantity, if a predetermined ultrafiltration rate is reached, and to issue a message for a user informing about the limitation of the substitution quantity.

9. The device according one of the preceding claims, wherein all necessary parameters can be predetermined by the user and the device is designed for reducing maximum quantities of the parameters preset by the user automatically, if this is necessary.

## Revendications

1. Dispositif pour le traitement extracorporel du sang, qui présente un dispositif de dialyse (8) et pour la réalisation d'une hémodialyse et d'une hémodiafiltration est conçu avec postdilution, comprenant :
- une interface utilisateur qui peut être commandée et/ou lue par un utilisateur, qui est agencée pour commencer le traitement du sang au moyen d'une hémodialyse en fonction de valeurs prédéfinies pour l'hémodialyse ;
- au moins une unité d'enregistrement et/ou de calcul, qui est agencée pour calculer des valeurs et/ou des rapports courants d'au moins un débit sanguin, d'une quantité d'ultrafiltrat, d'une quantité de substitution et/ou d'un type de substitution ;
- un dispositif de détection (6), qui est agencé pour enregistrer un progrès thérapeutique en fonction d'un signal de sortie d'un dispositif de détection (6) ;
- un dispositif de calcul de vitesses d'ultrafiltration, qui est agencé pour calculer une vitesse d'ultrafiltration dans le dispositif de dialyse (8) ; et
- un dispositif de régulation, qui est agencé pour réguler la quantité de substitution pendant l'hémodiafiltration avec postdilution,
**caractérisé en ce que**
le dispositif de traitement extracorporel du sang présente en outre :
- un dispositif de calcul de membrane secondaire, qui est agencé pour calculer une date de formation d'une membrane secondaire dans le dispositif de dialyse (8) en fonction de la vitesse d'ultrafiltration calculée ; et
- un dispositif de commutation, qui est agencé pour passer de l'hémodialyse à une hémodiafiltration avec postdilution après écoulement d'une période de temps prédéterminée, la période de temps prédéterminée étant une période de temps suivant un premier enregistrement d'un débit sanguin au niveau du dispositif de détection (6), et le passage de l'hémodialyse à l'hémodiafiltration avec postdilution étant effectué automatiquement, dans le cas où le dispositif de calcul de vitesses d'ultrafiltration après écoulement de la période de temps prédéterminée calcule une vitesse d'ultrafiltration prédéfinie, dans laquelle un débit de sang à traiter prédéterminé est considéré comme atteint et la membrane secondaire est considérée comme présente en tant qu'état de formation prédéterminé.

2. Dispositif selon la revendication 1, dans lequel la période de temps prédéterminée est initialement d'environ 3 à 5 minutes et le dispositif étant conçu pour adapter la période de temps prédéterminée après le début du traitement à une période de temps réelle en fonction des valeurs données et des valeurs actuellement calculées et pour démarrer automatiquement de manière différée l'hémodiafiltration avec postdilution.

3. Dispositif selon la revendication 1, dans lequel le dispositif de détection (6) est agencé pour effectuer le premier enregistrement du débit sanguin au moyen d'un capteur sanguin disposé sur un dispositif de détection d'air.

4. Dispositif selon l'une quelconque des revendications précédentes, dans le dispositif de régulation est agencé pour réguler la quantité de substitution en fonction d'une proportion maximale de substitution spécifique à un patient selon un rapport fixe pour le débit sanguin.

5. Dispositif selon l'une quelconque des revendications précédentes, dans lequel le dispositif de régulation est agencé pour réguler la quantité de substitution pour une taille ou une quantité constante.

6. Dispositif selon la revendication 4, dans lequel le rapport pour le débit sanguin s'élève à maximum 25 à 30 % du débit sanguin.

7. Dispositif selon l'une quelconque des revendications 4 à 6, dans lequel le dispositif de régulation est agencé pour diminuer automatiquement la quantité de substitution, si le débit sanguin passe en dessous d'une quantité minimale prédéterminée, et d'émettre un message notifiant à un utilisateur la diminution de la quantité de substitution.

8. Dispositif selon l'une quelconque des revendications précédentes, dans lequel le dispositif de régulation est agencé pour limiter automatiquement la quantité de substitution lorsqu'une vitesse d'ultrafiltration prédéfinie a été atteinte et émettre un message notifiant à un utilisateur la limitation de la quantité de substitution.

9. Dispositif selon l'une quelconque des revendications précédentes, dans lequel l'ensemble des paramètres nécessaires à l'utilisateur peut être prédéfini et le dispositif étant conçu pour diminuer automatiquement si nécessaire les quantités maximales des paramètres prédéfinis par l'utilisateur.
